# EUROPEAN PATENT SPECIFICATION

(11) **EP 3 409 217 B1**
(45) Date of publication and mention of the grant of the patent: **07.05.2025**
(21) Application number: 18174874.0
(22) Date of filing: 29.05.2018
(51) Int. Cl.: A61B 17/115, A61B 90/00, A61B 17/34, A61M 13/00, A61B 17/11, A61B 17/072, A61B 17/00

(54) **A CIRCULAR STAPLING APPARATUS WITH A DEVICE FOR INSUFFLATING A BODY LUMEN**
EINE KREISFÖRMIGEN HEFTVORRICHTUNG MIT EINER VORRICHTUNG ZUR INSUFFLATION VON KÖRPERHÖHLEN
APPAREIL D'AGRAFAGE CIRCULAIRE POUR UN DISPOSITIF D'INSUFFLATION D'UN ORGANE

(30) Priority: 30.05.2017 US 201715607986
(43) Date of publication of application: 05.12.2018
(73) Proprietor: Covidien LP, Mansfield, MA 02048 (US)
(72) Inventor: Penna, Christopher, Guilford, Connecticut 06437 (US); Vestweber, Boris, 50968 Koln (DE)
(74) Representative: Maschio & Soames IP Ltd

(56) References cited:
- EP-A2- 1 652 481
- EP-A2- 2 353 520
- EP-A2- 3 243 449
- DE-U1- 202011 101 205
- US-A- 5 395 030
- US-A1- 2004 217 146
- US-A1- 2010 001 037
- US-B1- 6 451 029

## Description

### CROSS-REFERENCE TO RELATED APPLICATIONS

This application is a continuation-in-part of U.S. Patent Application Serial No. 14/996,467, filed January 15, 2016, which is a continuation of U.S. Patent Application Serial No. 14/011,103, filed August 27, 2013, now U.S. Patent No. 9,265,503, which claims the benefit of, and priority to, U.S. Provisional Patent Application Serial No. 61/698,148, filed September 7, 2012.

In addition, this application is a continuation-in-part of U.S. Patent Application Serial No. 14/117,206, filed November 7, 2014, which is a national stage of PCT/EP11/03609, filed July 19, 2011, which claims the benefit of, and priority to, German Patent Application No. 102011 102 686.3, filed May 20, 2011.

### BACKGROUND

### Technical Field

The present disclosure relates to circular surgical staplers and channel guides for use therewith. More particularly, the present disclosure relates to circular surgical staplers and their methods of use including a shell assembly having a port in communication with a body lumen.

### Description of Related Art

Circular stapling apparatuses may be used in endoscopic procedures, laparoscopic procedures, or through natural body orifices, for fastening tissue. The circular stapling apparatuses may be powered or manually-operated and may include a tool assembly that is configured to operably couple to a distal end of an elongated member that extends from a handle assembly. The handle assembly may be reusable and the tool assembly may be disposable. The tool assembly may include an anvil assembly and a cartridge assembly that houses one or more fasteners therein.

In use, a circular stapling apparatus (or circular surgical staplers) may be used to reattach rectum portions that were previously transected. In this instance, a physician may insert a distal end (including an anvil assembly) of the circular stapling apparatus into a rectum of a patient and maneuver the distal end up the colonic tract of the patient toward the transected rectum portions. The physician may also insert the remainder of the circular stapling apparatus (including the cartridge assembly) through an incision and toward the transected rectum portions. The anvil and cartridge assemblies are approximated toward one another and staples are ejected from the cartridge assembly toward the anvil assembly to form the staples in tissue to affect an end-to-end anastomosis.

After the end-to-end anastomosis has been effected, the circular stapling apparatus is removed from the surgical site. The physician may use a sigmoidoscope or other suitable device to inspect the anastomosis for bleeding, patency, and/or blood perfusion. In certain instances, the physician uses the sigmoidoscope to introduce CO₂ into the colonic tract to check for leaks adjacent the anastomosis.

EP 3 243 449 A2, a document according to Article 54(3) EPC, shows an adapter assembly for connecting a handle assembly with a loading unit. It has a trocar assembly supported within an elongate case from which a magnetized trocar unit is extended.

US 2004/0217146 A1 relates to circular staplers for performing rectal mucosectomies and other surgical procedures. The circular staplers include a shaft having a proximal and a distal end, a cartridge assembly having a plurality of staples and operatively coupled to the distal end of the shaft, the cartridge assembly having a housing and a distal end defining an inner chamber, an anvil assembly operatively coupled to the cartridge assembly, and a channel adapted to communicate with a source of vacuum and the cartridge assembly to transmit a vacuum to the inner chamber of the cartridge assembly for drawing tissue into the inner chamber of the cartridge assembly.

While the aforementioned circular stapling apparatuses are suitable for their intended purposes, it may be beneficial to provide a circular stapling apparatus including a port to provide a passageway to the surgical site, as doing so may reduce the amount of instruments and/or incisions necessary to perform the surgical procedure. Additionally, it may be beneficial to provide a channel guide for use with a circular stapling apparatus, as doing so may reduce the amount of instruments and/or incisions necessary to perform the surgical procedure.

### SUMMARY

The invention is defined by the appended claims.

The present disclosure relates to an end effector for use with a circular stapling apparatus. The end effector includes a shell assembly, a port and a stepped lumen. The shell assembly is configured to support a cartridge assembly thereon. The shell assembly includes an outer wall. The port is disposed on the outer wall of the shell assembly. The stepped lumen is disposed in fluid communication with the port, and extends through the outer wall of the shell assembly.

In disclosed embodiments, the stepped lumen includes a first portion disposed in fluid communication with the port, and a second portion disposed in fluid communication with the first portion. The first portion is disposed at a first angle with respect to the second portion. It is disclosed that the first angle may be between about 120° and about 150°, or equal to about 135°.

It is further disclosed that the stepped lumen includes a third portion disposed in fluid communication with the second portion. The second portion is disposed at a second angle with respect to the third portion. It is disclosed that the second angle may be between about 120° and about 150°, or equal to about 135°. In disclosed embodiments, the first angle is equal to the second angle.

The present disclosure also relates to a method of performing a surgical procedure. The method comprises positioning a channel guide in engagement with a circular stapling apparatus such that an elongate passageway of the channel guide is disposed externally to and adjacent an elongated member of the circular stapling apparatus, aligning an aperture of a mounting portion of the channel guide with an opening of a shell assembly of the circular stapling apparatus, performing a surgical procedure with the circular stapling apparatus, and introducing at least one of a fluid or a device through the elongate passageway of the channel guide and through the aperture of the mounting portion of the channel guide.

In disclosed embodiments, positioning the channel guide in engagement with the circular stapling apparatus includes moving the mounting portion of the channel guide in a distal-to-proximal direction to mechanically engage the shell assembly of the circular stapling apparatus.

It is further disclosed that the method includes rotating a mounting portion of the channel guide with respect to the shell assembly while the channel guide is engaged with the circular stapling apparatus.

Additionally, it is disclosed that introducing at least one of a fluid or a device through the aperture of the mounting portion includes delivering fluid distally through the elongate passageway of the channel guide, through the aperture, and to a surgical site.

In disclosed embodiments, introducing at least one of a fluid or a device through the aperture of the mounting portion includes removing fluid from a surgical site through the aperture of the channel guide and proximally through the elongate passageway of the channel guide.

It is further disclosed that introducing at least one of a fluid or a device through the aperture of the mounting portion includes advancing a camera distally through the elongate passageway of the channel guide, through the aperture, and to a surgical site.

Additionally, it is disclosed that introducing at least one of a fluid or a device includes introducing at least one of a guide wire or an endoscopic camera through the elongate passageway of the channel guide, through the aperture, and to a surgical site.

In aspects of the present disclosure, a method of inserting a circular stapling apparatus into a body lumen includes inserting a distal portion of a shell assembly of the circular stapling apparatus in an entrance to a body lumen, introducing an insufflation fluid through an aperture defined in an outer surface of the shell assembly, and inserting a remainder of the shell assembly and a portion of an elongated member of the circular stapling apparatus into the body lumen. Introducing the insufflation fluid through the aperture flows insufflation fluid into the body lumen.

In aspects, introducing the insufflation fluid through the aperture includes inserting a hose through the port such that the hose extends from within the shell assembly and into the body lumen. Introducing the insufflation fluid through the aperture may include the insufflation fluid being a liquid that lubricates the body lumen. Additionally or alternatively, introducing the insufflation fluid through the aperture may include increasing a volume of the body lumen.

In some aspects, the method includes engaging a channel guide with the aperture of the shell assembly. Engaging the channel guide with the aperture of the shell assembly may include positioning a mounting portion of the channel guide about the shell assembly. Introducing the insufflation fluid through the aperture includes introducing the insufflation fluid through a passageway that is defined by the channel guide. The passageway may be in fluid communication with the aperture.

In certain aspects, engaging the engaging the channel guide with the aperture of the shell assembly includes inserting a nipple of the channel guide into the aperture of the shell assembly. Inserting the nipple of the channel guide into the aperture may include securing the nipple to the shell assembly with tabs that extend from the nipple. Engaging the channel guide with the aperture of the shell assembly may occur after inserting the distal portion of the shell assembly of the circular stapling apparatus into a body lumen.

In particular aspects, engaging the channel guide with the aperture of the shell assembly includes moving the mounting portion of the channel guide in a distal-to proximal direction to mechanically engage the shell assembly. The method may include rotating the mounting portion of the channel guide with respect to the shell assembly while the channel guide is engaged with the circular stapling apparatus. The method may include advancing a camera distally through the channel guide, through the aperture, and to a surgical site. Additionally or alternatively, the method may include advancing a guide wire through the channel guide, through the aperture, and to a surgical site.

In another aspect of the present disclosure, a method of removing a circular stapling apparatus from a body lumen includes withdrawing a distal portion of a shell assembly of a circular stapling apparatus from a body lumen and introducing an insufflation fluid through an aperture defined in an outer surface of the shell assembly such that the insufflation fluid flows into the body lumen while withdrawing the distal portion.

In aspects, introducing the insufflation fluid through the aperture includes supplying the insufflation fluid through a hose in communication with the port such that the hose extends from within the shell assembly into the body lumen. Introducing the insufflation fluid through the aperture may include the insufflation fluid being a liquid which lubricates the body lumen. Introducing the insufflation fluid through the aperture may include increasing a volume of the body lumen.

Further, to the extent consistent, any of the aspects and/or embodiments described herein may be used in conjunction with any or all of the other aspects described herein.

### BRIEF DESCRIPTION OF THE DRAWING

Various embodiments of the present disclosure are described hereinbelow with references to the drawings, wherein:
FIG. 1 is a perspective view of a powered circular stapling apparatus including a channel guide engaged therewith, and with a distal end of the circular stapling apparatus disposed within tissue, in accordance with embodiments of the present disclosure;
FIG. 2 is a perspective view of a manually operated circular stapling apparatus including the channel guide engaged therewith, and with a distal end of the circular stapling apparatus disposed within tissue, in accordance with embodiments of the present disclosure;
FIG. 3 is a perspective view of a shell assembly of a circular stapling apparatus including a port, shown partially inserted within a tissue lumen;
FIG. 4 is a partial, cut-away view of the shell assembly shown in FIG. 3 shown with fluid being introduced through the port and into the tissue lumen;
FIG. 5 is a partial, cut-away view of the shell assembly shown in FIG. 3 shown with an endoscopic camera extending through the port and within the tissue lumen;
FIG. 6 is a perspective view of a portion of the channel guide coupled to the shell assembly of the circular stapling apparatus shown in FIG. 1, according to embodiments of the present disclosure;
FIG. 7 is a cross-sectional view of the portion of the channel guide and the circular stapling apparatus shown in FIG. 6;
FIG. 8 is the indicated area of detail shown in FIG. 7;
FIG. 9 is a side view of a distal portion of the circular stapling apparatus shown in FIG. 6 engaged with a portion of the channel guide
FIG. 10 is a perspective view of a circular stapling apparatus including an irrigation tube, in accordance with embodiments of the present disclosure;
FIG. 11 is an exploded view with parts separated of the irrigation tube of FIG. 10;
FIG. 12 in an enlarged view of the shell assembly of the circular stapling apparatus of FIG. 10 with the irrigation tube positioned adjacent a port of the shell assembly;
FIG. 13 is a cross-sectional view taken along the section line 13-13 of FIG. 10;
FIG. 14 is a cross-sectional view taken along the section line 14-14 of FIG. 10; and
FIG. 15 is a cross-sectional view taken along section line 15-15 of FIG. 10.

### DETAILED DESCRIPTION

Particular embodiments of the present circular surgical staplers will be described herein with reference to the accompanying figures. As shown in the figures and as described throughout the following description, and as is traditional when referring to relative positioning on an object, the term "proximal" refers to the portion of the device that is closer to the user and the term "distal" refers to the portion of the device that is farther from the user. In the following description, well-known functions or constructions are not described in detail to avoid obscuring the present disclosure in unnecessary detail.

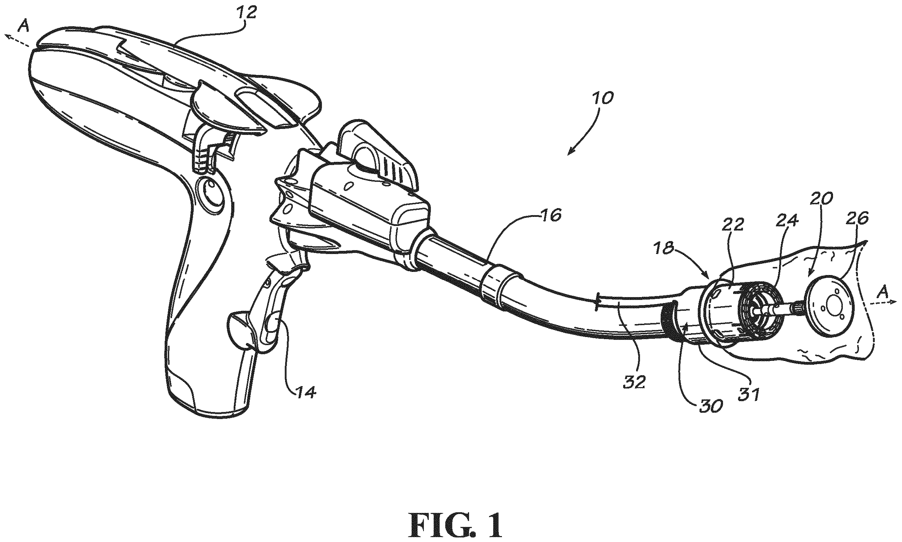

FIG. 1 illustrates a powered circular stapling apparatus 10 in accordance with embodiments of the present disclosure. Generally, circular stapling apparatus 10 includes a housing or handle assembly 12 having an actuator 14, and an elongated member 16 extending distally from handle assembly 12. In the illustrated embodiment, elongated member 16 is curved. However, it is envisioned that the elongated member 16 may be linear to suit a particular surgical procedure, e.g., mucosectomy, hemorrhoidectomy, etc. A tool assembly 18 (e.g., a multi-use loading unit, or a single-use loading unit) is coupled to or is configured to operably couple to a distal end of elongated member 16 and includes an end effector 20. In disclosed embodiments, a proximal portion of the tool assembly 18 is formed as a single component with the elongated member 16.

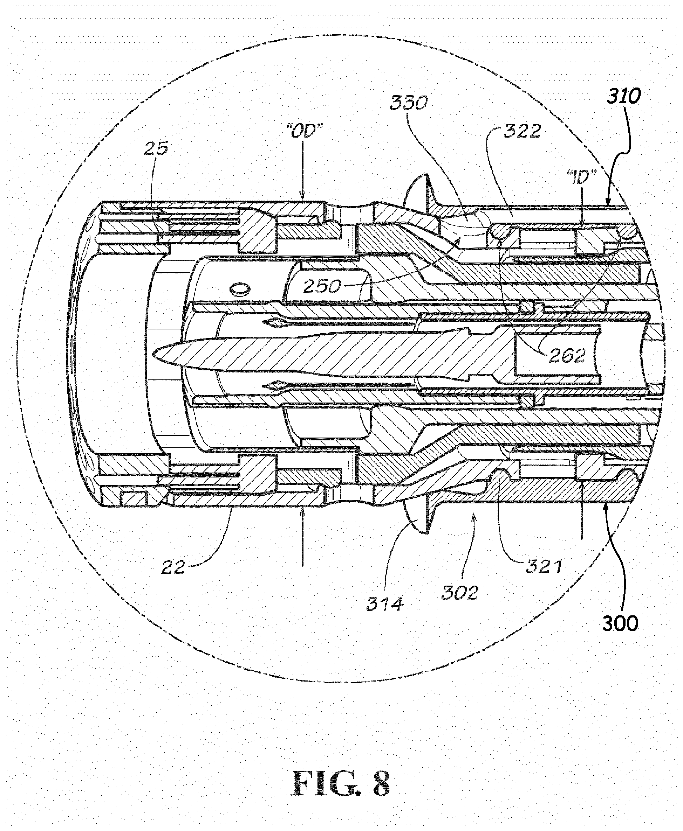

End effector 20 includes a shell assembly 22 that is configured to support a cartridge assembly 24 thereon. Cartridge assembly 24 is configured to house a plurality of fasteners (not shown) and includes a corresponding plurality of pusher members 25 (FIG. 8) that are operatively engagable with the fasteners to eject the fasteners from the cartridge assembly 24. While fasteners are not shown in the accompanying figures, it is known in the art to include fasteners within a cartridge assembly. As such, the present disclosure includes a cartridge assembly 24 with a plurality of fasteners housed therein.

End effector 20 also includes an anvil assembly 26 that is supported to move in relation to the cartridge assembly 24 between spaced and approximated positions. Anvil assembly 26 includes a plurality of pockets or depressions (not explicitly shown) that are each configured to receive and deform a fastener when the fasteners are deployed from cartridge assembly 24. Additionally, a channel guide 30 including a mounting portion 31 and an elongate passageway 32 is coupled to the shell assembly 22 as described in further detail below.

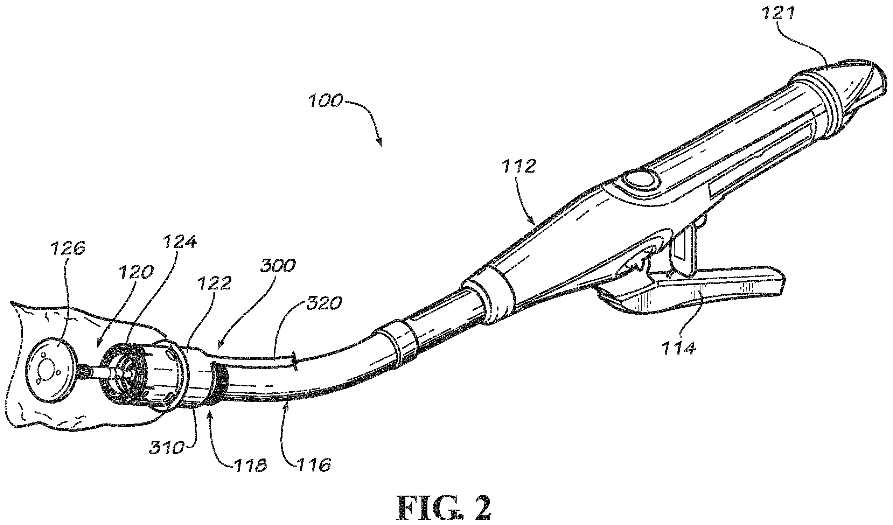

FIG. 2 illustrates a manually operated circular stapling apparatus 100 in accordance with embodiments of the present disclosure. Circular stapling apparatus 100 includes a handle assembly 112, an elongated member 116 extending distally therefrom, a tool assembly 118, which may be removably or fixedly coupled to the elongated member 116, and an end effector 120. The elongated body 116 extends distally from a distal end portion of the handle assembly 112 to a proximal end portion of the tool assembly 118. End effector 120 includes a shell assembly 122 that is configured to support a cartridge assembly 124 thereon, and an anvil assembly 126. Cartridge assembly 124 is configured to house a plurality of fasteners (not shown) and includes a corresponding plurality of pusher members that are substantially identical to pusher members 25 (FIG.8) and which are operatively engagable with the fasteners (not shown). Anvil assembly 126 is supported to move in relation to cartridge assembly 124 between spaced and approximated positions and includes a plurality of pockets or depressions (not explicitly shown) that are configured to receive and deform corresponding fasteners when the fasteners are deployed from cartridge assembly 124. The handle assembly 112 includes a rotatable advancing member 121 for longitudinally moving anvil assembly 126 with respect to cartridge assembly 124 and a pivotable trigger member 114 for ejecting fasteners from cartridge assembly 124. Additionally, a channel guide 300 including a mounting portion 310 and an elongate passageway 320 is coupled to the shell assembly 122.

Further details of circular stapling apparatuses are disclosed in U.S. Patent Application Publication No. 2014/0252062 filed on February 21, 2014, U.S. Patent Application Publication No. 2014/0197225, filed on January 11, 2013, U.S. Patent No. 8,806,973and U.S. Patent No. 9,010,609

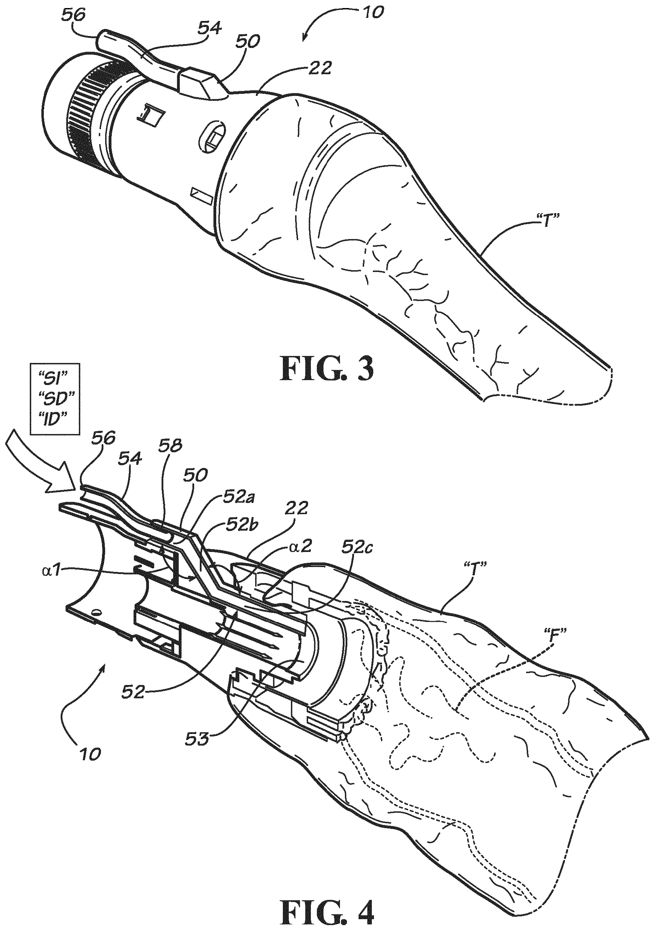

With particular reference to FIGS. 3-5, embodiments of circular stapling apparatus 10 are shown. Additionally, while circular stapling apparatus 100 is not explicitly shown in FIGS. 3-5, the features disclosed in FIGS. 3-5 are also applicable to the embodiments of circular stapling apparatus 100. Here, instead of channel guide 30, shell assembly 22 includes a port 50 disposed thereon. Port 50 is formed on an exterior wall of the shell assembly 22 and is configured to receive an elongated and/or flexible instrument (e.g., an endoscopic camera "EC," a tube associated with an irrigation device "ID" and/or a suction device "SD, etc.) therethrough, such that a portion of the elongated instrument can access the surgical site. Other surgical instruments that may be received in the port 50 for passage to the surgical site may include, but are not limited to, endoscopic graspers, endoscopic forceps, and endoscopic electrosurgical devices, which are shown schematically as "SI" in FIG. 4. The port 50 also provides an inlet for fluid "F" through the shell assembly 22 to a surgical site, and an outlet for fluid from the surgical site though the shell assembly 22, as will be described in detail below.

The port 50 is in fluid communication with a lumen 52 defined or secured within the shell assembly 22. The lumen 52 has a proximal end that is in fluid communication with port 50 and a distal, open end that may be positioned adjacent a distal end of a circular knife 53, for example, which is provided in the shell assembly 22 to sever stapled tissue sections. As shown in FIG. 4, the lumen 52 includes a first or proximal portion 52a, a second or intermediate portion 52b, and a third or distal portion 52c, which together define lumen 52 having a stepped or non-linear configuration. That is, the proximal portion 52a is disposed at an angle α1 with respect to the intermediate portion 52b, and the intermediate portion 52b is disposed at an angle α2 with respect to the distal portion 52c. It is envisioned that angle α1 may be between about 120° and about 150°; in embodiments angle α1 may be equal to about 135°. It is envisioned that angle α2 may be between about 120° and about 150°; in embodiments angle α2 may be equal to about 135°. It is further envisioned that angle α1 may be equal to or different from angle α2. Alternatively, other lumen configurations are envisioned.

A guide channel in the form of a hose 54 is couplable to the port 50 of the shell assembly 22. The hose 54 may provide a passageway to the port 50 for a surgical instrument being inserted through the port 50, e.g., an irrigation device "ID" or suction device "SD." In the embodiment illustrated in FIGS. 3-5, the hose 54 is formed from a material that is relatively flexible (e.g., plastic, rubber, etc.). Alternatively, the hose 54 may be formed from a material that is relatively rigid (e.g. plastic, metal, etc.).

In embodiments, the hose 54 extends longitudinally at least partially along the elongated member 16, 116 (FIGS. 1 and 2) and may be fixedly secured to a portion of the circular stapling apparatus 10 (e.g., the shell assembly 22, the elongated member 16, and/or the handle assembly 12) via one or more suitable securement methods (e.g., low tack adhesives, clips, bands, press- or friction-fit, etc.), not explicitly shown. It is further envisioned that hose 54 can be any reasonable length and may extend beyond the circular stapling apparatus 10.

A distal portion 58 (FIG. 4) of the hose 54 may be coupled to the port 50 via one or more suitable coupling methods, e.g., adhesive, welding, etc. In embodiments, the distal portion 58 of the hose may be removably coupled to the port 50 such that the port 50 may receive different types of hoses and/or different sizes of hoses. In this instance, the port 50 and the distal portion 58 of the hose 54 may couple to one another via a press- or friction-fit connection, a mechanical interface, or other suitable coupling methods. Alternatively, hose 54 may slidingly engage port 50 without any connection therebetween.

With particular reference to FIG. 4, the hose 54 includes a proximal end 56 that is configured to engage an irrigation device "ID," a suction device "SD," and/or another surgical instrument "SI," which are shown schematically in FIG. 4 as a single unit. Such irrigation/suction devices are known in the art and, therefore, are not described in further detail.

In embodiments, after an anastomosis has been created, the irrigation device "ID" can be coupled to the port 50 or the proximal end 56 of the hose 54 to circulate one or more suitable fluids "F" (e.g., saline, CO₂, etc.) through the hose 54 and/or port 50, and through the lumen 52 to the anastomosis site. As described above, introducing such fluids "F" into the colonic tract, for example, can be useful to check for leaks adjacent the anastomosis. The suction device "SD" may be coupled to the port 50 or the proximal end 56 of the hose 54 to remove fluid "F" from the anastomosis site, or to remove and/or suction other matter from the anastomosis site, e.g., tissue, etc.

In some embodiments, fluids "F" can be introduced to the anastomosis site during insertion of the stapling apparatus 10. By introducing fluids "F" into the anastomosis site during insertion of the stapling apparatus 10, the volume of the tissue lumen "T" can be increased to facilitate an injury-free insertion of the stapling apparatus 10 to the anastomosis site. Additionally or alternatively, introducing fluids "F" into the anastomosis site may lubricate the tissue lumen "T" to facilitate friction-free insertion of the stapling apparatus 10.

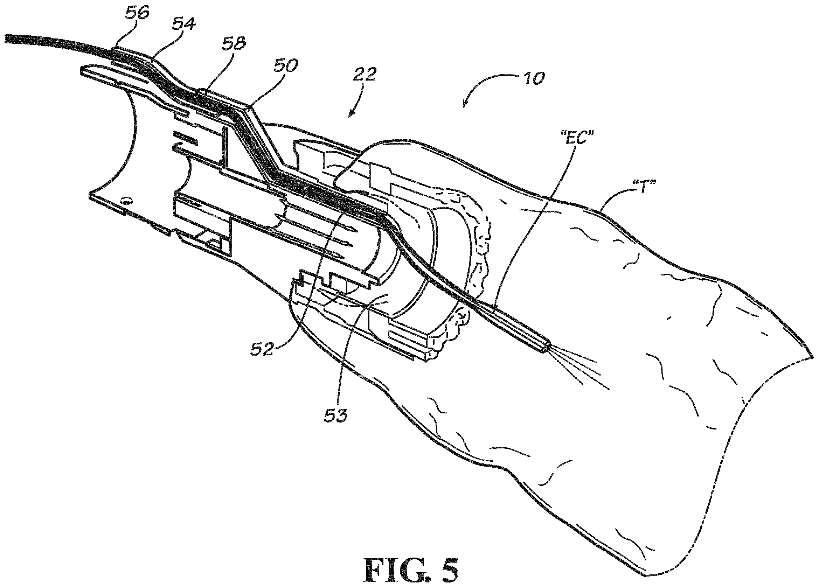

Additionally, other devices may be coupled to or extend through port 50. Such devices include endoscopic cameras "EC", guide wires, etc. Referring to FIG. 5, endoscopic camera "EC" is shown extending through hose 54 and port 50, and is within a tissue lumen "T." Endoscopic cameras are known in the art and, therefore, are not described herein in further detail. In embodiments, before and/or after an anastomosis has been created, a distal end of the endoscopic camera "EC" may be inserted through the hose 54 and/or port 50 of the shell assembly 22, and through the lumen 52 to the anastomosis site, for example. As described above, a user may use the endoscopic camera "EC" to inspect the anastomosis for bleeding, patency, and/or blood perfusion, for instance.

Additionally, it is envisioned that the stepped nature of lumen 52 helps maintain surgical instruments "SI" at a desired position within tissue. For example, when a flexible surgical instrument "SI" is inserted through the stepped lumen 52 such that a distal end of the surgical instrument "SI" is within tissue, the surgical instrument "SI" is less likely to move proximally with respect to the shell assembly 22 because of the non-linear path of the stepped lumen 52. That is, a surgical instrument "SI" will be physically hindered as it travels through a stepped lumen 52. By contrast, if the lumen 52 were linear, movement of fluid within the body tissue, contact between the surgical instrument "SI" and tissue, etc. may cause the surgical instrument "SI" to freely translate proximally or distally through such a linear lumen and thus not be optimally positioned.

Although only one port 50 is shown on the shell assembly 22 (FIG. 5), it is envisioned that the shell assembly 22 may include a plurality of ports 50 that are positioned on the exterior surface thereof. In these embodiments, each port 50 may be in fluid communication with the lumen 52 defined in the shell assembly 22. Alternatively, each port 50 may be in fluid communication with a corresponding lumen of a plurality of lumens 52 defined in the shell assembly 22. In such embodiments, for example, the suction device "SD" can be coupled to a first port, and an endoscopic camera "EC" can be coupled to a second port.

In embodiments, the port 50 may extend longitudinally at least partially along the tool assembly 18 and/or the elongated member 16 of the circular stapling apparatus 10. Additionally, the port 50 may be monolithically formed with the tool assembly 18 and/or the elongated member 16. As can be appreciated, in such embodiments, hose 54 may be omitted.

It is envisioned that the lumen 52 can extend from the shell assembly 22 to the handle portion 12 (e.g., to a proximal end of the handle portion 12). In such embodiments, the port 50 is positioned on the handle portion 12 and allows endoscopic camera "EC," irrigation device "ID," and/or suction device "SD" to be inserted from a location adjacent the handle portion 12, through at least a majority of the circular stapling apparatus 10, and into fluid communication with the anastomosis site, for example.

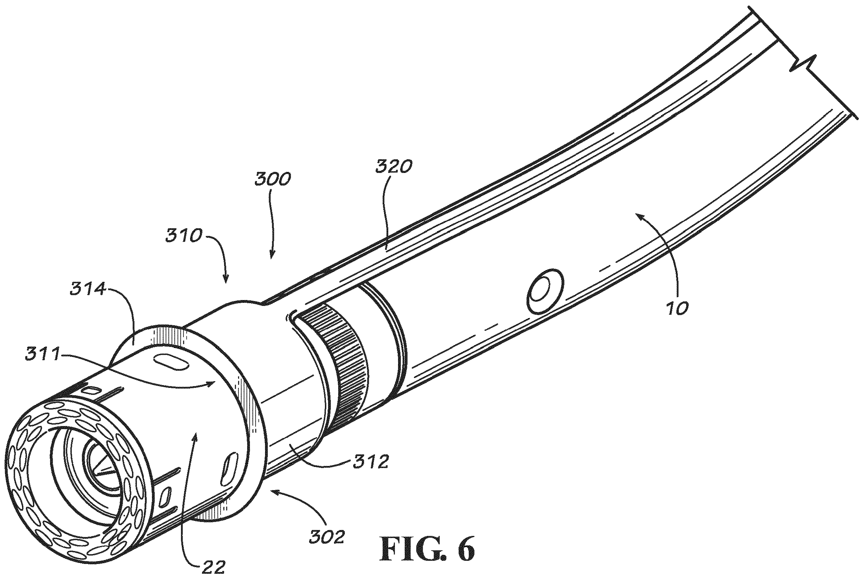

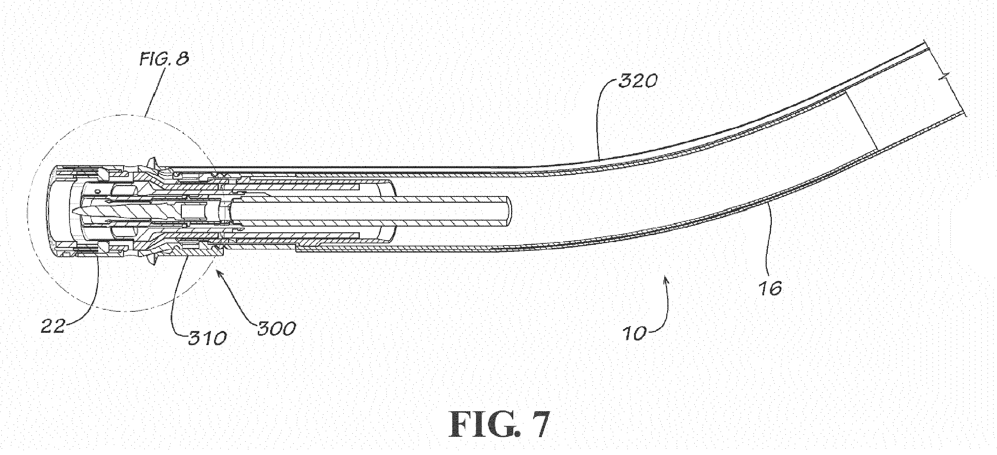

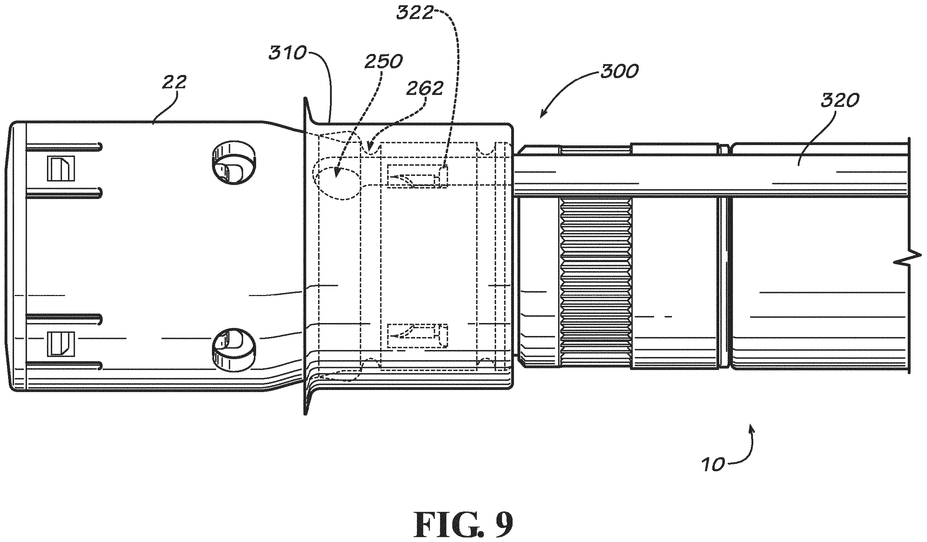

Referring to FIGS. 6-9 a channel guide 300 according to embodiments of the present disclosure is illustrated. In FIGS. 6-9, the channel guide 300 is shown coupled to the circular stapling apparatus 10. Channel guide 300 is configured to engage (either fixedly or removably) shell assembly 22 of circular stapling apparatus 10.

Channel guide 300 includes a mounting portion 310 and an elongate passageway 320 extending proximally therefrom. Mounting portion 310, which defines a longitudinal aperture 311, is sized and configured to mechanically engage shell assembly 22 of circular stapling apparatus 10.

More particularly, shell assembly 22 can be inserted (e.g., in a proximal-to-distal direction) at least partially through longitudinal aperture 311 of mounting portion 310. That is, mounting portion 310 can be positioned onto shell assembly 22 in a distal-to-proximal direction. In such embodiments, a distal portion 302 of channel guide 300 can flex radially outwardly to allow a portion of shell assembly 22 having a larger outer diameter "OD" than an inner diameter "ID" of channel guide 300 to be inserted into the mounting portion 310. Further, and with particular reference to FIG. 8, channel guide 300 includes inwardly depending lip(s) or rib 321 (e.g., annular lips 321) configured to be received within recess(es) 262 (e.g., annular recesses 262) of shell assembly 22. It is envisioned that the receipt of between lip(s) 321 within recess(es) 262 helps longitudinally align and sealingly secure the channel guide 300 to the shell assembly 22. It is further envisioned that alternatively or in addition to lip(s) 321 and recess(es) 262, channel guide 300 may be attached to shell assembly 22 with an adhesive.

It is also disclosed to mechanically engage channel guide 300 and circular stapling instrument 10 by first inserting elongated member 16 through longitudinal aperture 311 of mounting portion 310, then engaging shell assembly 22 with a distal end of elongated member 16, and then moving mounting portion 310 distally with respect to shell assembly 22. Here, too, lip(s) 321 is received within recess(es) 262 to help longitudinally align and sealingly secure the channel guide 300 to shell assembly 22.

Mounting portion 310 includes a body 312 defining an aperture 330 and a lumen 322 therein. Aperture 330 extends through a wall of body 312 and is disposed in fluid communication with lumen 322, which extends through body 312 and through elongate passageway 320 of channel guide 300. Additionally, aperture 330 of mounting portion 310 is configured to align with an opening 250 extending through an outer wall of shell assembly 22 that is in fluid communication with an interior space of shell assembly 22. Thus, the lumen 322 of elongate passageway 320 of channel guide 300 is in fluid communication with opening 250 of shell assembly 22, and with the interior space of shell assembly 22. In disclosed embodiments, as shown in FIGS. 1 and 2, elongate passageway 320 is a tube that extends externally of and parallel to elongate body 116. A proximal end of elongate passageway 320 may be disposed proximally of handle assembly 112, aligned with a portion of handle assembly 112, or disposed distally or handle assembly 112. As can be appreciated, devices (e.g., an endoscopic camera "EC") and/or fluid "F" can travel through a proximal end of elongate passageway 320, into the interior space of shell assembly 22, and to the surgical site.

It is envisioned that aperture 330 extends around a portion (e.g., majority or entirety) of a perimeter of mounting portion 310, thus facilitating radial alignment between aperture 330 and opening 250 of shell assembly 22. Additionally, the engagement between annular lip(s) 321 and annular recess(es) 262 may facilitate and/or enable the relative rotation of channel guide 300 with respect to shell assembly 22, e.g., to help align aperture 330 with opening 250 to provide a pathway to the surgical site.

In the illustrated embodiments, a distal end of mounting portion 310 includes a flange 314 extending along an outer periphery thereof. It is envisioned that flange 314 provides a surface for a user to grasp to facilitate coupling the channel guide 300 to the shell assembly 22, for example. While flange 314 is shown on a distal part of mounting portion 310, it is envisioned that flange 314 can be disposed on a different part of mounting portion 310 (e.g., on a proximal part thereof), and that mounting portion 310 can include more than one flange 314 thereon. For instance, it is envisioned that mounting portion 310 includes a first flange 314 on a proximal part thereof, and a second flange 314 on a distal part thereof.

It is further envisioned that channel guide 300 includes a seal member or grommet disposed in mechanical cooperation with aperture 330 and/or with a portion of lumen 322 to help seal or otherwise contain air or fluid pressurizations within tissue and/or to prevent spillage of such fluid.

Methods of using circular stapling apparatus 10, 100 and/or channel guide 300, as described above, are also disclosed herein. Additionally, the present disclosure includes methods of coupling channel guide 300 with circular stapling apparatus 10, 100, as disclosed herein.

For example, disclosed methods of performing a surgical procedure include selectively engaging channel guide 300 with circular stapling apparatus 10, aligning aperture 330 of channel guide 300 with opening 250 of shell assembly 22, performing a surgical procedure with circular stapling apparatus 10, and transporting at least one of a fluid "F" or a device (e.g., an endoscopic camera "EC") through aperture 330 of channel guide 300.

Additionally, embodiments of the disclosed methods include positioning channel guide 300 in engagement with circular stapling apparatus 10 such that elongate passageway 320 of channel guide 300 is disposed externally to and adjacent elongated member 16 of circular stapling apparatus 10, aligning an aperture 311 of mounting portion 310 of channel guide 300 with opening 250 of shell assembly 22 of circular stapling apparatus 10, performing a surgical procedure with circular stapling apparatus 10, and introducing at least one of a fluid "F" or surgical instrument "SI" through elongate passageway 320 of channel guide 300 and through aperture 311 of mounting portion 310 of channel guide 300.

Further aspects of the methods include positioning channel guide 300 in a distal-to-proximal direction to mechanically engage shell assembly 22 of circular stapling apparatus 10, and rotating mounting portion 310 of channel guide 300 with respect to shell assembly 22 while channel guide 300 is engaged with circular stapling apparatus 10. It is envisioned that rotation of mounting portion 310 with respect to the shell assembly 22 helps orient a surgical instrument "SI" in a desired location within tissue with respect to the shell assembly 22, for example.

It is envisioned that channel guide 300 is usable with various different types of surgical instruments, including those that include a replaceable cartridge assembly 24. It is further envisioned that channel guide 300 can be engaged and disengaged with circular stapling apparatus 10, or that circular stapling apparatus 10 is manufactured to include channel guide 300 already engaged therewith.

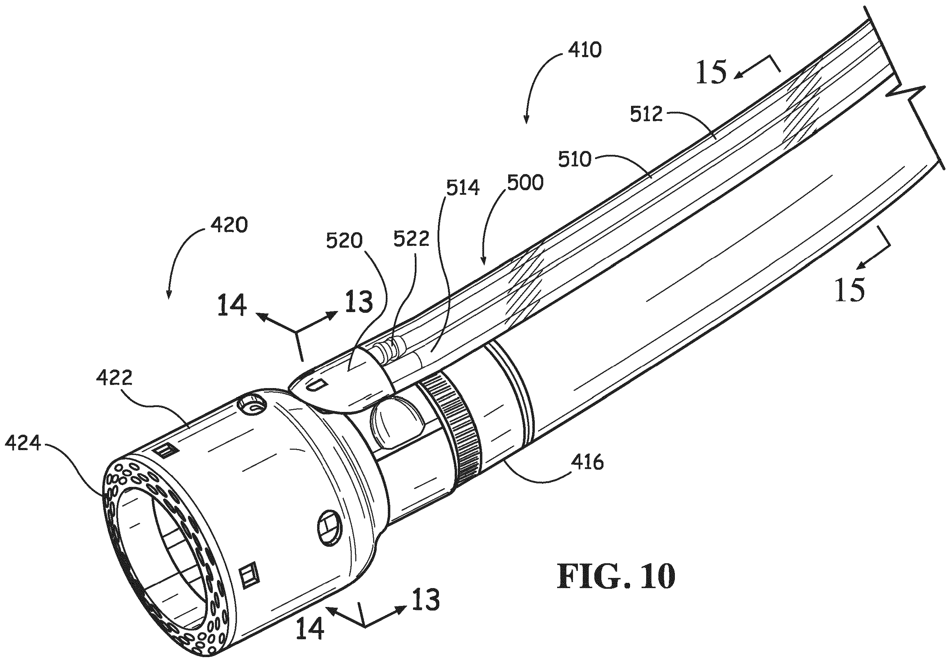

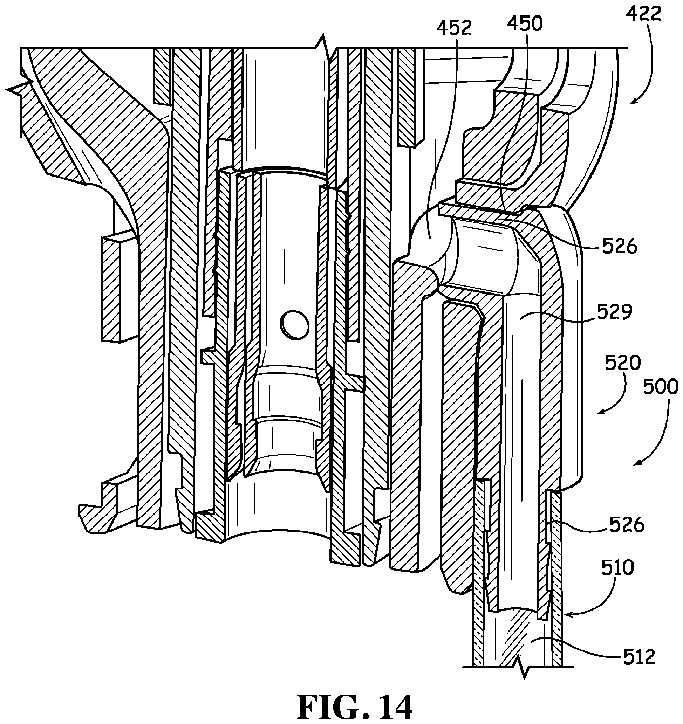

Referring to FIGS. 10-14, embodiments of a circular stapling apparatus 410 are shown. The circular stapling apparatus 410 is similar to the circular stapling apparatus 10 detailed above with similar structures represented with reference numerals including a "4" preceding the previous reference numeral. Additionally, while circular stapling apparatuses 10 and 100 are not explicitly shown in FIGS. 10-14, the features disclosed in FIGS. 10-14 are also applicable to the embodiments of circular stapling apparatuses 10 and 100. With particular reference to FIG. 10, the circular stapling apparatus 410 includes an end effector 420 including a shell assembly 422 that is releasably coupled to an elongate member 416, and a guide channel or irrigation tube 500 that is coupled to the shell assembly 422.

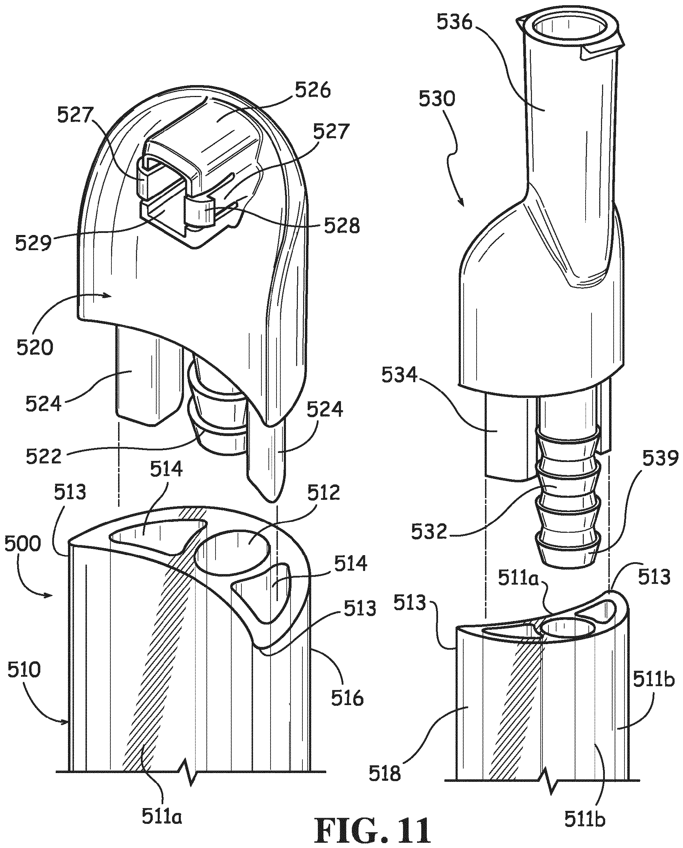

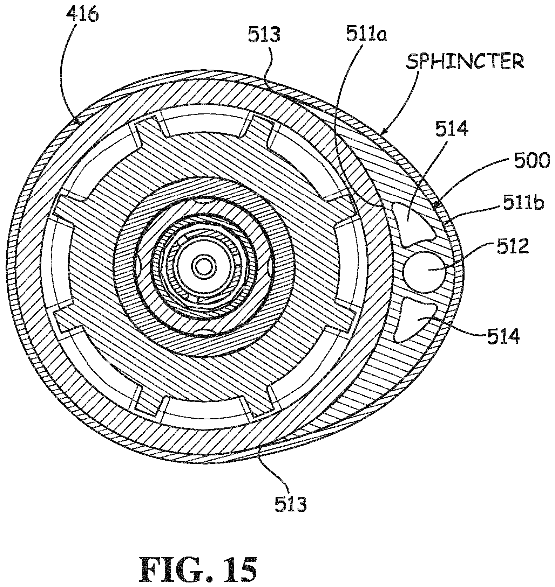

Referring to FIG. 11, the irrigation tube 500 includes a flexible tube 510, a distal coupling 520, and a proximal coupling 530. The flexible tube 510 has a distal portion 516 and a proximal portion 518 and defines a central lumen 512 and two channels 514 positioned on either side of the central lumen 512 between distal and proximal portions 516, 518. The flexible tube 510 may be constructed from a clear plastic that is extruded to form the flexible tube 510. The flexible tube 510 has an inner surface 511a and an outer surface 511b. The inner surface 511a has a radius substantially equal to a radius of an outer surface of the elongate member 416 (FIG. 10) of circular stapling apparatus 410 such that the inner surface 511a is in substantial contact with the elongate member 416 when the distal coupling 520 is secured to the shell assembly 422 as detailed below. With reference momentarily to FIG. 15, the surface profile of inner surface 511a (e.g., the radius of curvature) is such that application of fluid/moisture (F) to inner surface 511a of irrigation tube 500 helps to adhere irrigation tube to elongate member 416 of circular stapling apparatus 410 by way of a suctioning effect.

The outer surface 511b of the flexible tube 510 has a radius slightly less than the radius of the inner surface 511a such that the outer surface 511b meets the inner surface 511a at edges 513. The outer surface 511b is shaped such that the outer surface 511b forms a smooth transition with the outer surface of the elongate member 416 when the distal coupling 520 is secured to the shell assembly 422 as detailed below. With reference to FIG. 15, the smooth transition between the outer surface 511b of the flexible tube 510 and the outer surface of the elongate member 416 allows a sphincter (S) to form a seal about the elongate member 416 with the irrigation tube 500 attached. Specifically, the smooth transition eliminates gaps between the flexible tube 510 and the elongate member 416 which may prevent a sphincter from fully sealing about the elongate member 416 when the irrigation tube 500 is attached, for example, as is the case with other irrigation tubes having transverse cross-sectional profiles that are circular or of other shape.

The distal coupling 520 includes a central connector 522 that extends proximally into the central lumen 512 defined by the flexible tube 510. The central connector 522 may include ribs that engage walls defining the central lumen 512 to prevent the distal coupling 520 from separating from flexible tube 510. The distal coupling 520 also includes one or more proximally extending protrusions 524 (FIG. 11) that are each received within one of the passages 514 of the flexible tube 510 to secure the distal coupling 520 with the flexible tube 510. Each protrusion 524 may be shaped to conform to the shape of a respective passage 514. The distal coupling 520 also includes a nipple 526 that is substantially orthogonal to the central connector 522. The nipple 526 and the central connector 522 define a coupler lumen 529 that is in fluid communication with the central lumen 512 of the flexible tube 510 when the central connector 522 is received within the central lumen 512. The nipple 526 also includes tabs 527 on opposite sides of the nipple 526 and includes outer detents 528 that are configured to engage the shell assembly 422 to couple the distal coupling 520 to the shell assembly 422 as detailed below. The tabs 527 are resilient and biased outward.

The proximal coupling 530 includes a central connector 532 that extends distally into the central lumen 512 defined by the flexible tube 510. The central connector 532 may include ribs that engage walls defining the central lumen 512 to prevent the proximal coupling 530 from separating from flexible tube 510. The proximal coupling 530 also includes one or more distally extending protrusions 534 that are received within one of the passages 514 of the flexible tube 510 to secure the proximal coupling 530 with the flexible tube 510. Each protrusion 524 may be shaped to conform to the shape of a respective passage 514. The proximal coupling 530 includes a proximally extending connection 536 that is configured to couple to a fluid source. As shown, the proximally extending connection 536 is a male luer connector but it is contemplated that the proximally extending connection 536 may be a female luer connector or another known fluid coupling device. The proximally extending connection 536 and the central connector 532 define a coupler lumen 539 that is in fluid communication with the central lumen 512 when the central connector 532 is received within the central lumen 512.

It is contemplated that the protrusions 524 and 534 may define openings in fluid communication with the respective passages 514 and that the proximal coupling 530 and the distal coupling 520 may include additional connectors to fluidly connect the passages with a fluid source and to the circular stapling apparatus 410. In addition, the nipple 526 and/or the proximally extending connection 536 may be segmented such that a single connection may provide three distinct fluid channels between the distal and proximal couplings 520, 530. It is envisioned that each of the channels may provide a fluid to or withdraw a fluid from the circular stapling apparatus 410.

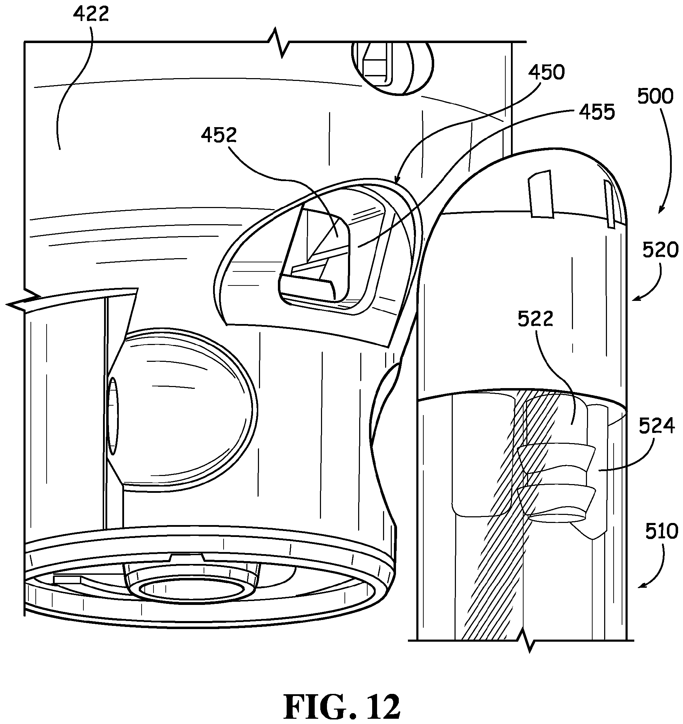

Referring now to FIGS. 12-14, the shell assembly 422 defines an opening or port 450 that is in fluid communication with a lumen 452 defined within the shell assembly 422. The lumen 452 passes through the shell assembly 422 such that the port 450 is in fluid communication with an anastomosis site through the lumen 452. The port 450 is defined by walls 455 that extend inward from an outer surface of the shell assembly 422.

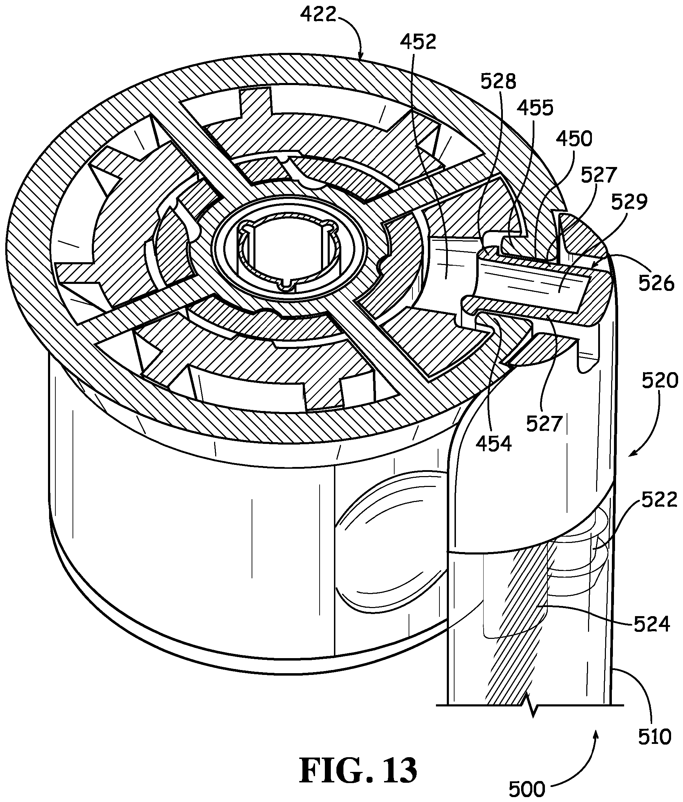

With reference to FIG. 13, the nipple 526 of the distal coupling 520 of the irrigation tube 500 is coupled to the port 450 of the shell assembly 422. As the nipple 526 is inserted into the port 450, the detents 528 engage the walls 454 such that the tabs 527 are urged inwardly as the nipple 526 enters the port 450. When the detents 528 extend past the walls 455, the resilience of the tabs 527 snap the detents 528 outward to engage an inner surface of the walls 455 to couple the distal coupling 520 to the port 450. When the distal coupling 520 is coupled to the port 450, the nipple 526 seals the port 450 such that fluid may flow into the lumen 452 of the shell assembly 422 from the central lumen 512 of the irrigation tube 500 and/or from the lumen 452 of the shell assembly 422 into the central lumen 512 of the irrigation tube 500. As shown, when the distal coupling 520 is coupled to the shell assembly 422, the irrigation tube 500 is non-removably attached to the shell assembly 422. However, it is contemplated that the irrigation tube 500 may include a release mechanism (not shown) that allows the distal coupling 520 to releasably couple to the shell assembly 422.

With particular reference to FIGS. 3-5, a method of inserting the stapling apparatus 10 into a tissue lumen "T" in accordance with the present disclosure is disclosed. While detailed herein below with reference to apparatus 10, it will be appreciated that the method may also be used with stapling apparatuses 100 and 410. Initially, the stapling apparatus 10 is brought into close proximity with the tissue lumen "T" such that the shell assembly 22 can be inserted into an end of the tissue lumen "T" as shown in FIG. 3.

With the shell assembly 22 positioned within the end of the tissue lumen "T", an insufflation fluid "F" is introduced through the port 50 defined in the shell assembly 22. The insufflation fluid "F" can be a liquid, e.g., saline, that lubricates the tissue lumen "T" and increases the volume of the tissue lumen "T" to facilitate atraumatic insertion of the shell assembly 22 into the tissue lumen "T". When the tissue lumen "T" is insufflated to an appropriate degree, the stapling apparatus 10 can be advanced to the anastomosis site as detailed above.

After the stapling apparatus 10 is advanced to the anastomosis site, the stapling apparatus 10 is fired. After the stapling apparatus 10 is fired, the stapling apparatus 10 is removed from the patient. It has been shown that additional insufflation fluid "F" can be introduced through the port 50 as the stapling apparatus 10 is removed from the patient to assist in removing the stapling apparatus 10 from the patient. Specifically, the additional insufflation fluid "F" may expand the tissue lumen "T" and/or lubricate the tissue lumen "T" to assist in removing the stapling apparatus 10 from the tissue lumen "T".

The irrigation tube 500 may be provided as a part of a kit with a surgical stapling apparatus, e.g., surgical stapling apparatus 10, 100, 410.

From the foregoing and with reference to the various figure drawings, those skilled in the art will appreciate that certain modifications can also be made to the present surgical stapling apparatuses without departing from the scope of the same. While several embodiments of surgical stapling apparatuses have been shown in the drawings, it is not intended that the disclosure be limited thereto Therefore, the above description should not be construed as limiting, but merely as exemplifications of particular embodiments. Those skilled in the art will envision other modifications within the scope of the claims appended hereto.

## Claims

1. A circular stapling apparatus comprising:
an end effector including a shell assembly configured to support a cartridge assembly thereon said cartridge assembly configured to house a plurality of fasteners, and comprising a distal portion, wherein said distal portion is configured to be inserted into an entrance to a body lumen;
an aperture defined in an outer surface of the shell assembly in fluid communication with a lumen of said shell assembly ; and
an elongated member releasably coupled from the shell assembly, further comprising a channel guide, wherein the channel guide defines a passageway, said passageway configured to be in fluid communication with the aperture,
**characterised in that**
the channel guide further comprises a nipple configured for insertion into the aperture of the shell assembly, wherein said nipple is secured to the shell assembly via tabs extending form the nipple.

2. The apparatus (410) according to claim 1 wherein the tabs (527) are resilient and biased outward.

3. The apparatus (410) according to claim 1 wherein the guide channel includes and irrigation tube comprising a flexible tube, a distal coupling and a proximal coupling.

4. The apparatus (410) according to claim 3 wherein flexible tube defining a central lumen (512) with two channels (514) positioned on either side of the central lumen (512) between the distal and proximal portions (516, 518).

5. The apparatus (410) according to claim 3 or any claim dependent thereon, wherein the flexible tube (510) has an inner surface (511a) and an outer surface (511b), wherein the inner surface (511a) has a radius substantially equal to a radius of an outer surface of the elongate member (416) of circular stapling apparatus (410) such that the inner surface (511a) is in substantial contact with the elongate member (416) when the distal coupling (520) is secured to the shell assembly (422).

6. The apparatus (410) according to claim 3 or any claim dependent thereon, wherein the distal coupling (520) includes a central connector (522) that extends proximally into the central lumen (512) defined by the flexible tube (510).

7. The apparatus (410) according to claim 6, wherein the central connector (522) includes ribs that engage walls defining the central lumen (512) to prevent the distal coupling (520) from separating from flexible tube (510).

8. The apparatus (410) according to claim 7, wherein the distal coupling (520) including one or more proximally extending protrusions (524) that are each configured to be received within one of the passages (514) of the flexible tube (510) to secure the distal coupling (520) with the flexible tube (510).

9. The apparatus (410) according to claim 3 or any claim dependent thereon, wherein the proximal coupling (530) includes a central connector (532) that extends distally into the central lumen (512) defined by the flexible tube (510), the proximal coupling including one or more distally extending protrusions (534) that are received within one of the passages (514) of the flexible tube (510) to secure the proximal coupling (530) with the flexible tube (510).

10. A circular stapling apparatus of claim 1 wherein said aperture (330) extends through a wall of said body (312) and is disposed in fluid communication with lumen (322).

11. The circular stapling apparatus according to claim 1, further comprising a hose adapted to be inserted through the aperture such that the hose extends from within the shell assembly into the body lumen during use.

## Patentansprüche

1. Kreisförmiges Klammerinstrument, umfassend:
einen Endeffektor, der eine Schalenanordnung aufweist, die dazu ausgelegt ist, eine Magazinanordnung daran zu tragen, wobei die Magazinanordnung dazu ausgelegt ist, eine Vielzahl von Befestigungselementen zu beherbergen, und der einen distalen Abschnitt umfasst, wobei der distale Abschnitt dazu ausgelegt ist, in einen Eingang zu einem Körperlumen eingesetzt zu werden;
eine Öffnung, die in einer Außenseite der Schalenanordnung in Fluidverbindung mit einem Lumen der Schalenanordnung definiert ist; und
ein längliches Element, das lösbar mit der Schalenanordnung gekoppelt ist, ferner umfassend eine Kanalführung, wobei die Kanalführung einen Durchgang definiert, wobei der Durchgang dazu ausgelegt ist, mit der Öffnung in Fluidverbindung zu sein, **dadurch gekennzeichnet, dass** die Kanalführung ferner einen Nippel umfasst, der zum Einsetzen in die Öffnung der Schalenanordnung ausgelegt ist, wobei der Nippel über Zungen, die sich von dem Nippel erstrecken, an der Schalenanordnung befestigt ist.

2. Vorrichtung (410) nach Anspruch 1, wobei die Zungen (527) elastisch und nach außen vorgespannt sind.

3. Vorrichtung (410) nach Anspruch 1, wobei der Führungskanal ein Spülrohr aufweist, das ein flexibles Rohr, eine distale Kopplung und eine proximale Kopplung umfasst.

4. Vorrichtung (410) nach Anspruch 3, wobei das flexible Rohr ein zentrales Lumen (512) mit zwei Kanälen (514) definiert, die auf beiden Seiten des zentralen Lumens (512) zwischen dem distalen und dem proximalen Abschnitt (516, 518) positioniert sind.

5. Vorrichtung (410) nach Anspruch 3 oder einem davon abhängigen Anspruch, wobei das flexible Rohr (510) eine Innenfläche (511a) und eine Außenfläche (511b) hat, wobei die Innenfläche (511a) einen Radius hat, der im Wesentlichen einem Radius einer Außenfläche des länglichen Elements (416) der kreisförmigen Klammervorrichtung (410) gleicht, sodass die Innenfläche (511a) in dauerhaftem Kontakt mit dem länglichen Element (416) ist, wenn die distale Kopplung (520) an der Schalenanordnung (422) befestigt ist.

6. Vorrichtung (410) nach Anspruch 3 oder einem davon abhängigen Anspruch, wobei die distale Kopplung (520) einen zentralen Verbinder (522) aufweist, der sich proximal in das zentrale Lumen (512) erstreckt, das von dem flexiblen Rohr (510) definiert wird.

7. Vorrichtung (410) nach Anspruch 6, wobei der zentrale Verbinder (522) Rippen aufweist, die Wände in Eingriff nehmen, die das zentrale Lumen (512) definieren, um zu verhindern, dass sich die distale Kopplung (520) von dem flexiblen Rohr (510) trennt.

8. Vorrichtung (410) nach Anspruch 7, wobei die distale Kopplung (520) einen oder mehrere sich proximal erstreckende Vorsprünge (524) aufweist, die jeweils dazu ausgelegt sind, in einen der Durchgänge (514) des flexiblen Rohrs (510) aufgenommen zu werden, um die distale Kopplung (520) an dem flexiblen Rohr (510) zu befestigen.

9. Vorrichtung (410) nach Anspruch 3 oder einem davon abhängigen Anspruch, wobei die proximale Kopplung (530) einen zentralen Verbinder (532) aufweist, der sich distal in das zentrale Lumen (512) erstreckt, das von dem flexiblen Rohr (510) definiert wird, wobei die proximale Kopplung einen oder mehrere sich distal erstreckende Vorsprünge (534) aufweist, die in einem der Durchgänge (514) des flexiblen Rohrs (510) aufgenommen werden, um die proximale Kopplung (530) in dem flexiblen Rohr (510) zu befestigen.

10. Kreisförmige Klammervorrichtung nach Anspruch 1, wobei sich die Öffnung (330) durch eine Wand des Körpers (312) erstreckt und mit dem Lumen (322) in Fluidverbindung angeordnet ist.

11. Kreisförmige Klammervorrichtung nach Anspruch 1, ferner umfassend einen Schlauch, der dazu angepasst ist, derart durch die Öffnung eingesetzt zu werden, dass sich der Schlauch während der Verwendung aus der Schalenanordnung in das Körperlumen erstreckt.

## Revendications

1. Appareil d'agrafage circulaire comprenant :
un effecteur terminal comportant un ensemble coque configuré pour supporter un ensemble cartouche sur celui-ci, ledit ensemble cartouche étant configuré pour loger une pluralité d'attaches, et comprenant une partie distale, dans lequel ladite partie distale est configurée pour être insérée dans une entrée d'une lumière corporelle ;
une ouverture définie dans une surface externe de l'ensemble coque en communication fluidique avec une lumière dudit ensemble coque ; et
un élément allongé accouplé de manière libérable depuis l'ensemble coque, comprenant en outre un guide de canal, dans lequel le guide de canal définit une voie de passage, ladite voie de passage étant configurée pour être en communication fluidique avec l'ouverture, **caractérisé en ce que** le guide de canal comprend en outre un mamelon configuré pour insertion dans l'ouverture de l'ensemble coque, ledit mamelon étant assujetti à l'ensemble coque par l'intermédiaire de languettes s'étendant depuis le mamelon.

2. Appareil (410) selon la revendication 1 dans lequel les languettes (527) sont élastiques et
sollicitées vers l'extérieur.

3. Appareil (410) selon la revendication 1 dans lequel le canal de guidage comprend un tube d'irrigation comprenant un tube flexible, un accouplement distal et un accouplement proximal.

4. Appareil (410) selon la revendication 3 dans lequel le tube flexible définit une lumière centrale (512) avec deux canaux (514) positionnés de chaque côté de la lumière centrale (512) entre les parties distale et proximale (516, 518).

5. Appareil (410) selon la revendication 3 ou une quelconque revendication dépendant de celle-ci, dans lequel le tube flexible (510) a une surface interne (511a) et une surface externe (511b), dans lequel la surface interne (511a) a un rayon sensiblement égal à un rayon d'une surface externe de l'élément allongé (416) de l'appareil d'agrafage circulaire (410) de telle sorte que la surface interne (511a) soit en contact important avec l'élément allongé (416) lorsque l'accouplement distal (520) est assujetti à l'ensemble coque (422).

6. Appareil (410) selon la revendication 3 ou une quelconque revendication dépendant de celle-ci, dans lequel l'accouplement distal (520) comporte un raccord central (522) qui s'étend proximalement dans la lumière centrale (512) définie par le tube flexible (510).

7. Appareil (410) selon la revendication 6, dans lequel le raccord central (522) comporte des nervures qui entrent en prise avec des parois définissant la lumière centrale (512) pour empêcher l'accouplement distal (520) de se séparer du tube flexible (510).

8. Appareil (410) selon la revendication 7, dans lequel l'accouplement distal (520) comporte une ou plusieurs protubérances (524) s'étendant proximalement qui sont chacune configurées pour être reçues à l'intérieur de l'un des passages (514) du tube flexible (510) pour assujettir l'accouplement distal (520) avec le tube flexible (510).

9. Appareil (410) selon la revendication 3 ou une quelconque revendication dépendant de celle-ci, dans lequel l'accouplement proximal (530) comporte un raccord central (532) qui s'étend distalement dans la lumière centrale (512) définie par le tube flexible (510), l'accouplement proximal comportant une ou plusieurs protubérances (534) s'étendant distalement qui sont reçues dans l'un des passages (514) du tube flexible (510) pour assujettir l'accouplement proximal (530) avec le tube flexible (510).

10. Appareil d'agrafage circulaire selon la revendication 1 dans lequel ladite ouverture (330) s'étend à travers une paroi dudit corps (312) et est disposée en communication fluidique avec la lumière (322).

11. Appareil d'agrafage circulaire selon la revendication 1, comprenant en outre un tuyau conçu pour être inséré à travers l'ouverture de telle sorte que le tuyau s'étende depuis l'intérieur de l'ensemble coque dans la lumière corporelle pendant l'utilisation.
